# EUROPÄISCHE PATENTSCHRIFT

(11) **EP 1 089 708 B1**
(45) Veröffentlichungstag und Bekanntmachung des Hinweises auf die Patenterteilung: **10.04.2002**
(21) Anmeldenummer: 99932722.4
(22) Anmeldetag: 24.06.1999
(51) Int. Cl.: A61K 7/48

(54) **VERWENDUNG VON MINDESTENS EINEM, AUS DEM KERN DER FRUCHT DES MIRABELLENBAUMS GEWONNENEN LIPIDEXTRAKT**
USE OF AT LEAST ONE LIPID EXTRACT OBTAINED FROM THE FRUIT SEED OF THE MIRABELLE TREE
UTILISATION D'AU MOINS UN EXTRAIT LIPIDIQUE OBTENU DANS LE NOYAU DU FRUIT DU MIRABELLIER

(30) Priorität: 25.06.1998 FR 9808186
(43) Veröffentlichungstag der Anmeldung: 11.04.2001
(73) Patentinhaber: Cognis France S.A., 31360 Saint Martory (FR)
(72) Erfinder: PAULY, Gilles, F-54000 Nancy (FR)
(74) Vertreter: Nuss, Pierre
(86) Internationale Anmeldenummer: EP9904383
(87) Internationale Veröffentlichungsnummer: WO9966899

(56) Entgegenhaltungen:
- WO-A-83/01898
- FR-A- 1 448 975
- FR-A- 2 610 197
- CHEMICAL ABSTRACTS, vol. 103, no. 15, 14. Oktober 1985 (1985-10-14) Columbus, Ohio, US; abstract no. 121966y, G. VERNIN ET AL: "Mirabelle plum aroma. Study by CG-SM (gas chromatography-mass spectrometry) SPECMA bank analysis" Seite 604; XP002102437 & PARFUMS, COSMET., AROMES, Bd. 62, 1985, Seiten 69-74,

## Beschreibung

Die vorliegende Erfindung betrifft das Gebiet der Kosmetologie, besonders Anwendungen auf Haut (kutane) und Haar (kapillare) und hat als Zielsetzung die Verwendung von mindestens einem Lipidextrakt aus dem Kern der Frucht des Mirabellenbaums oder der Mirabelle in der Kosmetik, sowie kosmetische Erzeugnisse (Kosmetika), die mindestens einen derartigen Extrakt enthalten.

Die Mirabelle ist die Frucht eines Baums (Mirabellenbaum), der zu einer sehr weitverbreiteten Gattung (Prunustyp, Rosaceaefamilie) gehört, zu der besonders die Mandel (prunus amygdalus) und folgende essbare Früchte gehören: die Aprikose (prunus armeniaca), die Kirsche (prunus avium, prunus cerasus), der Pfirsich (prunus persica), die Pflaumen (prunus domestica, eine Gattung, die viele Untergattungen beinhaltet). Die exakte botanische Bezeichnung der Pflanze ist Prunus, der Untergattung Punophora, der Gattung Insitisia, der Varietät Mirabelle.

Der Anbau des Mirabellenbaums, der im nordöstlichen Frankreich seit der Zeit der Römer stattfand, hat sich vom Mosel-Departement aus, besonders um die Stadt Metz entwickelt.

So wurde 1994 die Mirabellenernte in Frankreich auf sieben Millionen Tonnen geschätzt und die derzeitigen Hauptabsatzgebiete der Frucht sind Konservenfabriken (70 %), Brennereien (Destilleriegewerbe) (17 %), Frischobstkonsum (12 %).

Ein Vergleich hinsichtlich des Ölgehalts der Kerne der verschiedenen Früchte der Prunusgattung ausser der Mirabelle und ihrer Fettsäuren-Zusammensetzung ist in "Charakterisation of the seed oil and meal from apricot, cherry, nectarine, peach and plum" (Charakteristik des Öls und Pulvers aus Kernen von Aprikosen, Kirschen, Nektarinen, Pfirsichen und Pflaumen) Kamel B.S. & Kakuda Y., Journal of the American Oil Chemists' Society, 1992, Band 69, N° 5, S. 492 - 494 veröffentlicht worden.

Dieser Artikel ermöglicht besonders die Feststellung, dass aus Kernen und Mandeln dieser verschiedenen Früchte gewonnene Öle sehr naheliegende Fettsäuren-Zusammensetzungen haben und eine interessante Quelle für Ölsäuren (Oleinsäuren) (52 - 66 %) und Leinölsäuren (Linolsäuren) (28 - 35 %) bieten.

Ausserdem ist bekannt, dass Mandelkerne von essbaren Früchten der Prunusgattung Öle enthalten, von denen einige in der Kosmetik oder der Pharmakologie verwendet werden (süsse Mandel, Aprikose).

So kennt man aus der Schrift EP-A- 0 105 876 pharmazeutische Zusammensetzungen, die besonders ein Pflanzenöl der Prunusgattung enthalten, vorzugsweise Mandelöl und die zur Behandlung von Hauterkrankungen (dermatologischen Erkrankungen) verwendet werden.

Ausserdem betreffen die Schriften JP-A-01 313 414 und JP-A-01 038 014 haarwuchsfördernde Kosmetikmittel, die ausser Öl Extrakte von Pflanzen, darunter von prunus persica oder prunus armeniaca enthalten.

FR-A-2 610 197 offenbart ein kosmetisches Erzeugnis (Duschgel oder seife), welches einen nicht-alkoholischen Extrakt eines alkoholischen Getränkes, hergestellt aus Früchten der Prunusgattung (z.B. Mirabelle), enthält.

Aber keine Veröffentlichung, die dem Erfinder bekannt ist, betrifft die Anwendung von Extrakten aus Mirabellenkernen auf dem Gebiet der Kosmetik.

Der Erfinder hat jedoch festgestellt, dass aus Mirabellenkernen gewonnene Lipidextrakte über besondere Eigenschaften und eine sehr gute Toleranz verfügen, weshalb sie direkt in kosmetischen Erzeugnissen (Kosmetika) verwendet werden können.

Ausserdem bieten Mirabellenkerne, besonders in den Anbau- und Verarbeitungsgebieten dieser Früchte einen ausgiebigen und preiswerten Rohstoff, dessen Auswertung interessant ist.

Die Hauptzielsetzung der vorliegenden Erfindung liegt daher in der Verwendung von mindestens einem, aus den Kernen der Früchte des Mirabellenbaums gewonnenen Lipidextrakt, als Mittel zur Zubereitung eines kosmetischen Erzeugnisses zum örtlich wirkenden (topischen) Gebrauch auf dem Gebiet der Pflege der Haut, der Lippen, der Schleimhäute und/oder Phanere.

Nach einem ersten Merkmal der Erfindung besteht der Lipidextrakt aus ÖI oder aus einer oder mehreren Lipidfraktionen, die aus den Mandeln der Kerne der Früchte des Mirabellenbaums durch einen Vorgang entzogen wurde/n, der in der durch die Extraktion durch Warm- oder Kaltdruck, die Extraktion durch Lösungsmittel und die Extraktion durch superkritisches CO₂ gebildeten Gruppe gewählt wurde.

Mindestens der besagte Lipidextrakt kann dem kosmetischen Erzeugnis in roher, verfeinerter, desodorisierter oder verfeinerter und desodorisierter Form hinzugefügt werden.

Als Beispiele zur Veranschaulichung, aber nicht zur Einschränkung werden nachstehend zwei Vorgänge hinsichtlich der Ölgewinnung aus Mirabellenkernen beschrieben.

### Beispiel 1

Ungefähr 4,04 kg gereinigte und getrocknete, aus Konservenfabriken stammende Mirabellenkerne werden grob zerquetscht und die erreichte zerquetschte Masse wird gesiebt, um eine mit Mandelmehl angereicherte Fraktion zu erhalten: man erhält auf diese Weise 1,03 kg mit Mandeln angereichertes Mehl.

Das Mehl wird dann im Rücklauf in einem Reaktor durch 1,55 Liter Hexan extrahiert.

Nach dem Abkühlen bei Raumtemperatur wird der Hexanextrakt durch Filtern zurückgewonnen und dann durch einen rotativen Verdampfapparat (Evaporator) konzentriert, um eine ölige Fraktion zu erhalten, die bei 45° C in einen Trockenofen kommt, um die Spuren des Lösungsmittels zu beseitigen.

Man erhält auf diese Weise 105 ml leicht getrübtes, gelbes Rohöl, das einen angenehmen Mandelgeruch und einen 0,31 %igen Oleinsäuregehalt hat.

Der Ertrag hinsichtlich des Gewichts dieser Extraktion beträgt 2,60 % im Bezug auf die Kerne und 10,2 % im Bezug auf das mit Mandeln angereicherte Mehl.

### Beispiel 2

4,64 kg getrocknete, zerquetschte, aus Brennereien stammende Kerne werden in einem Reaktor und im Rücklauf eine Stunde lang mit 4,64 Liter Hexan extrahiert. Der Hexanextrakt wird dann wie im Beispiel 1 verarbeitet.

Man gewinnt auf diese Weise 298 ml trübes, dunkelgelbes Öl, das einen starken Geruch nach gegärten Früchten und einen hohen, bei 17,08 % im Gewicht liegenden Oleinsäuregehalt hat.

Dann wird das ÖI einem Verfeinerungs- und Desodorisierungsvorgang nach Techniken unterzogen, die dem Fachmann dieses Gebiets geläufig sind, um 169 ml gelbes, geruchloses ÖI mit einem 0,15 %igen Oleinsäuregehalt zu erreichen.

Die Gewichtserträge hinsichtlich des Öls betragen folglich 6,42 % Rohöl/Kerne und 3,6 % im Bezug auf das verfeinerte, desodorisierte ÖI.

Nach einer ersten Realisierungsmethode der Erfindung wird mindestens ein besagter, aus Mirabellenkernen gewonnener Lipidextrakt einem kosmetischen Erzeugnis für die Haut, die Lippen und/oder die Schleimhäute als weichmachendes, feuchtigkeitsspendendes Mittel hinzugefügt, das der Haut Frische und strahlenden Glanz verleiht.

Nach einer zweiten Realisierungsmethode der Erfindung wird mindestens ein besagter Lipidextrakt einem kosmetischen Erzeugnis für die Phanere, besonders für das Haar als weichmachendes, feuchtigkeitsspendendes Mittel hinzugefügt, um dem Haar Glanz zu verleihen und das Entwirren und Frisieren zu erleichtern.

Aber ausser den oben erwähnten Realisierungsmethoden kann/können der/die erwähnte/n Lipidextrakt/e auch als einziges oder nicht einziges aktives Mittel einem Erzeugnis oder einer kosmetischen Zusammensetzung hinzugefügt werden, für den örtlich wirkenden (topischen) gleichzeitigen Gebrauch für die Haut, die Lippen, die Schleimhäute und die Phanere.

Die vorteilhaften Eigenschaften der Lipidextrakte konnten durch verschiedene Tests, deren Durchführung und Ergebnisse nachstehend erklärt werden, bewiesen und mengenmässig bestimmt werden.

### Auswertung der weichmachenden und das Frisieren erleichternden Wirkung

Die Wirkung der Behandlung mit einem auf 1,5 % Mirabellenöl dosierten Shampoo, das nach dem Beispiel 2 zubereitet wurde, wird durch leichteres Frisieren und Entwirren von Menschenhaar im Vergleich zu einem Shampooplacebo quantitativ ausgewertet.

Das Prinzip der Methode besteht im Messen der Kraft, die sich dem Durchkämmen einer Haarsträhne bei einer konstanten Geschwindigkeit widersetzt.

Die ausgewerteten Parameter sind die maximale Kraft (N), die der aufgezeichneten Kraft während des Durchkämmens der Haarspitzen entspricht und die Arbeit (Nmm), die während des Frisierens ausgeführt wird, die der Fläche unter der Kurve Kraft-Verlängerung entspricht.

Das Messen wurde vor und nach der Behandlung mit dem Shampoo-placebo (5 Strähnen) oder mit dem auf 1,5 % Mirabellenöl dosierten Shampoo, (5 Strähnen) unter vollkommen identischen Voraussetzungen vorgenommen.

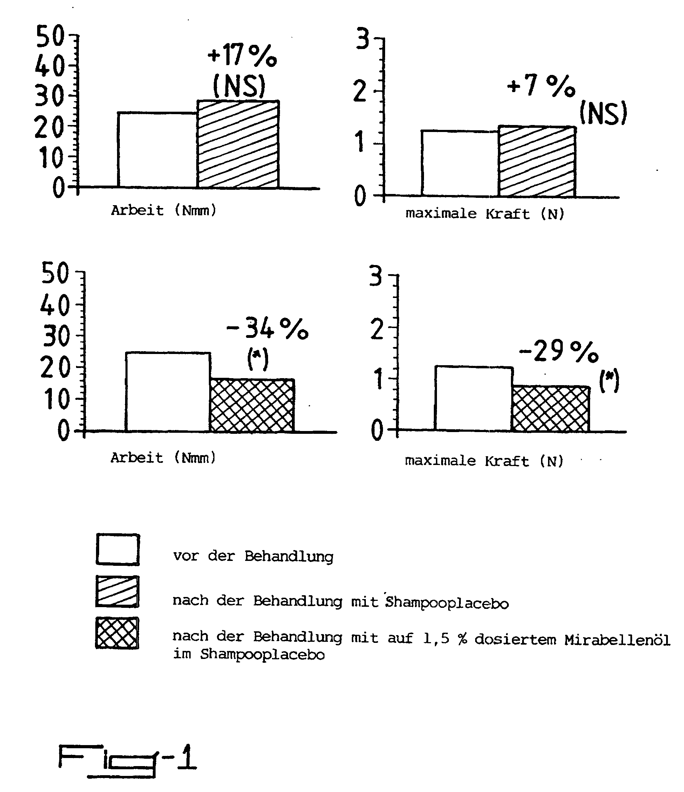

Die auf Figur 1 der beigefügten Zeichnungen dargestellten Ergebnisse (Wirkung auf die Eigenschaft des Haars leicht frisierbar zu sein: im Durchschnitt fünf Versuche +/- SEM/Test Wilcoxon bei paarweise zusammengestellten Serien) zeigen dass die Behandlung mit dem Shampooplacebo die Parameter der Frisierkraft nicht wesentlich (NS) beeinflusst.

Im Gegensatz dazu erzeugt die Behandlung mit dem auf 1,5 % Mirabellenöl dosierten Shampoo weicheres Haar, woraus sich eine 34 %ige Reduzierung (*:signifikativer Unterschied) der Arbeit während des Frisierens und eine 29 %ige Reduzierung (*: signifikativer Unterschied) der maximalen Kraft ergibt.

### Auswertung der weichmachenden Wirkung auf die Haut ex vivo

Die schmierstoffartige und weichmachende Wirkung der kosmetischen aktiven Mittel wirkt sich im biophysikalischen Aspekt durch eine verringerte Reibung zwischen der Haut und der mit ihr in Kontakt gebrachten Materie aus.

Die Reibungsreduzierung ist auf die Oberflächenveränderungen physikalischer Art zurückzuführen, deren Konsequenz weiche, geschmeidige und satinartige Haut ist.

Die weichmachende Wirkung des aus Mirabellenkernen gewonnenen Öls, das nach dem Beispiel 2 zubereitet wurde, wurde durch den Auswertungstest der Reibung auf der menschlichen Haut ex vivo im Vergleich zu einem Referenzerzeugnis (Eutanol G der Firma HENKEL) eine Stunde nach der Behandlung und durch einen Reibungsmesser (Friktiometer) des sogenannten veränderten Comaishtyps bewiesen.

Das Prinzip der Betriebsart eines derartigen Apparats ist folgendes: eine konstante Kraft wird auf die Haut durch einen Gleitschuh ausgeübt, der bei kontrollierter Geschwindigkeit und unter kontrollierten Drücken rotiert wird, wobei der Reibungsmoment gemessen wird und ermöglicht, den Reibungskoeffizienten des Gleitschuhs auf der Haut zu berechnen. Der Reibungskoeffizient hängt von der Oberflächenbeschaffenheit der Haut ab, besonders von ihrem Feuchtigkeitsgehalt und ihrer Weichheit.

Die Reibungen sind bei vier verschiedenen Rotationsgeschwindigkeiten gemessen worden, indem ein Standarddruck der Sonde auf die Haut ausgeübt wurde.

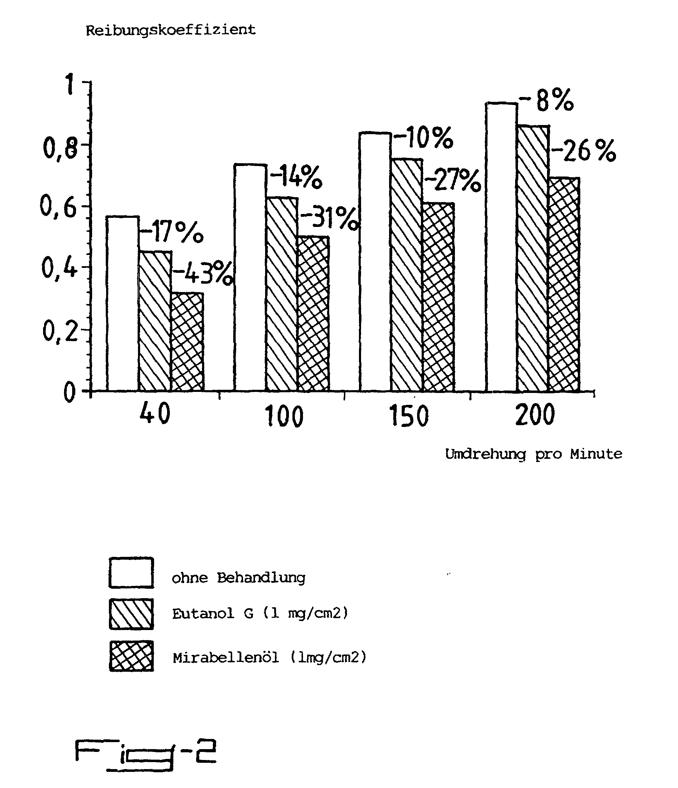

Die Ergebnisse werden auf Figur 2 dargestellt (Messdurchschnitte +/- SEM), die die Auswirkungen auf die Reibungseigenschaften der menschlichen Haut ex vivo darstellt, die Ergebnisse beweisen, dass die Behandlung mit Eutanol G eine leichte Verringerung der Reibung zwischen 17 % und 8 % je nach der Rotationsgeschwindigkeit hervorruft.

Die Verringerung der Reibung ist deutlich grösser (zwischen 43 % und 26 %) im Fall einer Behandlung mit Mirabellenöl.

### Auswertung des feuchtigkeitsspendenden Vermögens

Die feuchtigkeitsspendende Wirkung des nach den Beispielen 1 und 2 zubereiteten Mirabellenöls wurde durch Leitfähigkeitsmessung (Konduktometrie) 21 Stunden nach der standardisierten Anwendung (4 mg/cm²) auf der antero-internen Seite des Unterarms einer freiwilligen Person (Volontärs) gemessen: man stellt dann eine 15 %ig bis 20 %ige Zunahme der Hautfeuchtigkeit (kutane Hydratation) fest.

### Frische und strahlender Glanz des Teints

Diese Wirkungen sind durch Brillantometrie auf der antero-internen Seite des Unterarms 5 Minuten nach der standardisierten Anwendung (4 mg/cm²) des Öls ausgewertet worden: eine sehr gute glänzende Wirkung wurde festgestellt, die besser als die durch Referenzöl (Jojobaöl) mit einer geringen Verbesserung des Teints ist.

Die sensoriellen Tests auf der Haut beweisen auch dass es sich um sehr flächendeckendes Öl handelt, mit starkem Ausbreitungsvermögen, sehr filmbildend, das einen sehr glänzenden Film auf der Haut hinterlässt: dieser Film hält sich noch 15 Minuten nach der Anwendung, indem er ebenfalls ein sehr anhaltendes Gefühl der Weichheit gibt.

Diese verschiedenen Tests beweisen dass das Öl oder die Lipidextrakte des Kerns und ganz besonders der Mandel des Kerns der Mirabelle besondere Eigenschaften bieten, die sie für die Verwendung in kosmetischen Erzeugnissen (Kosmetika) nützlich machen, besonders für dermatologische Erzeugnisse, die für die Haut, die Lippen und Phanere, (besonders das Haar) bestimmt sind und besonders als weichmachendes, feuchtigkeits-, glanzspendendes Mittel, das den strahlenden Glanz der Haut verbessert und als Mittel, das das Frisieren erleichtert.

Die Zielsetzung der vorliegenden Erfindung ist ebenfalls ein kosmetisches Erzeugnis für den örtlich wirkenden (topische) Gebrauch für die Haut, die Schleimhäute und/oder die Lippen, dadurch gekennzeichnet dass es zwischen 0,05 % und 100 % im Gewicht, vorzugsweise zwischen 1 % und 10 % im Gewicht wenigstens einen aus der Mandel des Kerns der Frucht des Mirabellenbaums gewonnenen Lipidextrakt als aktives, weichmachendes, feuchtigkeitsspendendes Mittel enthält, das die Frische des Teints und dessen strahlenden Glanz verbessert.

Ausserdem betrifft die Erfindung ebenfalls ein kosmetisches Erzeugnis für den örtlich wirkenden (topischen) Gebrauch für Phanere, besonders für das Haar, dadurch gekennzeichnet dass es zwischen 0,05 % und 100 % im Gewicht und vorzugsweise zwischen 1 % und 10 % im Gewicht mindestens einen aus der Mandel des Kerns der Frucht des Mirabellenbaums gewonnenen Lipidextrakt als aktives, weichmachendes, feuchtigkeitsspendendes Mittel enthält, das den Glanz des Haars verbessert und sein Entwirren und Frisieren leichter macht.

Die vorab erwähnten Extrakte können für Anwendungen zur Pflege, Hygiene und zum Schutz der Haut (Erzeugnisse für Gesicht und Körper, Tag- und Nachtkosmetika, Sonnenschutzmittel, deckende Mittel, Mittel zum Schutz der Hände und Lippen), aber auch auf dem Gebiet der Pflege und der Behandlung des Haars (Haarwasser oder Shampoo, Cremes, Schaummittel, Schutzmittel, reparierende, weichmachende, filmbildende Mittel, sowie Lichtschutzmittel).

Als uneingeschränkte Beispiele für die praktischen Ausführungen der Erfindung werden nachstehend verschiedene kosmetische Erzeugnisse oder Zusammensetzungen beschrieben, die mindestens einen, aus Mirabellenkernen gewonnenen Lipidextrakt enthalten.

### Beispiel 1:

Ein kosmetisches Erzeugnis in Form eines feuchtigkeitsspendenden Mittels für trockenes Haar nach der Erfindung kann zum Beispiel eine Gewichtszusammensetzung aufweisen, die von den folgenden Fraktionen A und B aus gebildet ist, wie sie nachstehend beschrieben wird.

Fraktion A:
- Cetylalkohol 2,00 %
- Paraffinöl 2,00 %
- Sorbitanstearat 2,00 %
- Mirabellenöl (nach Beispiel N° 1 gewonnen) 2,00 %

Fraktion B:
- Glyzerin 2,00 %
- Laneth-20 1,00 %
- Cetrimoniumchlorid 2,00 %
- Konservierungsmittel qs
- destilliertes Wasser qsb 100,00 %

Der Vorgang für die Zubereitung und die Herstellung des oben erwähnten feuchtigkeitsspendenden Mittels für trockenes Haar besteht hauptsächlich darin, alle Bestandteile der Fraktion A (fette Phase) bei 75° unter Rühren schmelzen zu lassen, die Fraktion B (wasserhaltige Phase) auf 90° C zu erhitzen, indem die verschiedenen Bestandteile bei dieser Temperatur aufgelöst werden, die Fraktion A in die Fraktion B unter Rühren zu schütten und zuletzt das Gemisch unter Rühren abzukühlen, bis eine perfekte Homogenität erreicht wird.

### Beispiel 2:

Ein kosmetisches Erzeugnis in Form eines Shampoos zum Frisieren, Entwirren und Weichmachen nach der Erfindung kann zum Beispiel eine Gewichtszusammensetzung aufweisen, die von den folgenden Fraktionen A, B, C, D, E, F, G, H und I gebildet wird, wie sie nachstehend angegeben ist.

Fraktion A:
- Wasser 45,500 %
- Hydroxypropyl Guar Hydroxypropyltrimonioum-Chlorid 0,400 %

Fraktion B:
- Mischpolymerisat Acrylate/C10-30 Alkylakrylate 0,600 %
- Wasser 19,400 %

Franktion C:
- Natrium-Laurylsulfat (und) Lauryl-Polyglukose 25,000 %

Fraktion D:
- LS38 ATA-Stabilisator (serobiologische Labors) 0,300 %

Fraktion E:
- Titandioxid (und) Mika 0,600 %

Fraktion F:
- Triäthanolamin (20 %ige wässrige Lösung) 4,000 %

Fraktion G:
- Dimethicon 1,500 %
- Mirabellenöl (nach Beispiel N° 2 gewonnen) 2,000 %

Fraktion H:
- CG-Kathon (Octhilinone) 0,100 %

Fraktion I:
- Parfum 0,600%

Der Vorgang für die Zubereitung und Herstellung des oben erwähnten Shampoos besteht hauptsächlich in der Zubereitung der Fraktion A bei 82° C, im Hinzufügen der Fraktionen B, C und D zu A unter Turbinenrühren, danach im Hinzufügen der Fraktionen E und F unter Turbinenrühren danach im Abkühlen auf 30° C und zuletzt im Hinzufügen der Fraktionen G, H und I unter Planetenrühren.

### Beispiel 3:

Ein kosmetisches Erzeugnis in Form eines heilenden Haarbalsams für trockenes, glanzloses und strapaziertes Haar nach der Erfindung kann zum Beispiel eine Gewichtszusammensetzung aufweisen, die aus den folgenden Fraktionen A, B und C gebildet wird, wie sie nachstehend angegeben ist.

Fraktion A:
- Glyzerinstearat (und) PEG-100-Stearat 2,500 %
- Dimethicon 1,000 %
- Cyclomethicon 2,000 %
- Mirabellenöl (nach Beispiel N° 1 gewonnen) 3,000 %

Fraktion B:
- Wasser 85,800 %
- Elestab 388 (eingetragenes Warenzeichen) 2,500 %
- Guar-Hydroxypropyltrimonium-Chlorid 1,000 %
- Guargummi 1,000 %

Fraktion C:
- Natriumzitrat (20 %ige wässrige Lösung) 0,600 %
- Zitronensäure (10 %ige wässrige Lösung) 0,600 %

Der Vorgang für die Zubereitung und Herstellung des oben erwähnten Balsams besteht hauptsächlich in der Zubereitung der Fraktion A bei 75° C, in der separaten Zubereitung der Fraktion B bei 75° C unter Turbinenrühren, dem Hinzufügen der Fraktion A zur Fraktion B unter Turbinenrühren, dem Abkühlen des Gemischs auf 60° C unter Turbinenrühren, danach unter Planetenrühren und zuletzt bei Raumtemperatur, dem Einstellen des pH-Werts auf 6,0.

### Beispiel 4:

Ein kosmetisches Erzeugnis in Form eines Stäbchens für die Lippen (Lippenstift), um deren Glanz nach der Erfindung zu verstärken, kann zum Beispiel eine Gewichtszusammensetzung aufweisen, wie sie nachstehend angegeben ist:
- Rizinusöl 30,00 %
- Glyzerinstearat 43,00 %
- Isopropylmyristat 8,00 %
- Paraffinöl 5,00 %
- Mirabellenöl (nach Beispiel N° 2 gewonnen) 3,00%
- Vaseline 4,00 %
- Carnaubawachs 4,00 %
- Wollfettalkohol 3,00 %

Der Vorgang für die Zubereitung und die Herstellung des oben erwähnten Stäbchens für die Lippen (Lippenstift) besteht hauptsächlich in der Erhitzung aller Fettkörper unter Rühren auf 90° C, dem Ausschütten der geschmolzenen und homogenen Masse in eine Form und zuletzt dem Abkühlen und der Fertigstellung der Zubereitung nach klassischer Technik.

### Beispiel 5:

Ein kosmetisches Erzeugnis in Form eines Cremeöls in weichmachendem, feuchtigkeitsspendendem Wasser, das den Glanz der Haut nach der Erfindung erhöht, kann zum Beispiel eine Gewichtszusammensetzung aufweisen, die von den folgenden Fraktionen A, B und C gebildet wird, wie sie nachstehend angegeben ist.

Fraktion A:
- Triceteareth-4 Phospat 3,00%
- Polyglyzerin-2-PEG-4Stearat 8,00 %
- Paraffinöl 7,00 %
- Mirabellenöl (nach Beispiel N° 1 gewonnen) 5,00 %
- Isopropylpalmitat 8,00 %
- Stearyl-Heptanoat 2,00 %

Fraktion B:
- Methylparaben 0,20 %
- Glykolpropylen 2,00 %
- Imidazolidinyl-Karbonid 0,30 %
- Wasser 64,20 %

Fraktion C:
- Parfum 0,30%

Der Vorgang für die Zubereitung und Herstellung der oben erwähnten Creme besteht hauptsächlich im Erhitzen der Fraktion A auf 80° C unter Rühren, im Erhitzen der Fraktion B auf 70° C, dem Ausschütten der Fraktion A in die Fraktion B unter Turbinenrühren, danach im allmählichen Abkühlen unter Planetenrühren, danach im Hinzufügen der Fraktion C bei ungefähr 45° C und zuletzt im Anhalten des Rührens, nachdem wieder Raumtemperatur erreicht wurde.

Selbstverständlich ist die Erfindung nicht auf die beschriebenen und in den beiliegenden Zeichnungen dargestellten Realisierungsmethoden begrenzt. Änderungen sind möglich, besonders im Hinblick auf die Zusammensetzung der verschiedenen Elemente oder durch das Ersetzen technischer Äquivalente, ohne deshalb aus dem Schutzbereich der Erfindung zu geraten.

## Patentansprüche

1. Verwendung von mindestens einem, aus den Kernen der Früchte des Mirabellenbaums gewonnen Lipidextrakt als Mittel für die Zubereitung eines kosmetischen Erzeugnisses für den örtlich wirkenden (topischen) Gebrauch, für die Pflege der Haut, der Lippen, der Schleimhäute und/oder der Phanere.

2. Verwendung nach Anspruch 1, **dadurch gekennzeichnet dass** der Lipidextrakt aus Öl oder aus einer oder den Lipidfraktion/en, die aus den Mandeln der Kerne der Früchte des Mirabellenbaums durch einen Vorgang gewonnen wird/werden, der in der Gruppe ausgewählt wird, die durch die Extraktion unter Warm- oder Kaltdruck, der Extraktion durch ein Lösungsmittel und der Extraktion durch superkritisches CO₂ gebildet wird.

3. Verwendung nach Anspruch 1 oder 2, **dadurch gekennzeichnet dass** mindestens ein besagter Lipidextrakt dem kosmetischen Erzeugnis in roher, verfeinerter, desodorisierter oder verfeinerter und desodorisierter Form hinzugefügt wird.

4. Verwendung nach irgendeinem der Ansprüche 1 - 3, **dadurch gekennzeichnet, dass** mindestens ein besagter Lipidextrakt einem kosmetischen Erzeugnis für die Haut, die Lippen und/oder die Schleimhäute als Mittel zum Weichmachen, Feuchtigkeitsspenden und zur Verbesserung der Frische und des strahlenden Glanzes des Teints, hinzugefügt wird.

5. Verwendung nach irgendeinem der Ansprüche 1 bis 3, **dadurch gekennzeichnet, dass** mindestens ein besagter Lipidextrakt einem kosmetischen Erzeugnis für die Phanere und besonders für das Haar als Mittel zum Weichmachen, Feuchtigkeitsspenden und zur Verbesserung des Glanzes und für ein leichteres Entwirren und Frisieren des Haars hinzugefügt wird.

6. Kosmetisches Erzeugnis für den örtlich wirkenden (topischen) Gebrauch für die Haut, die Schleimhäute und/oder die Lippen, **dadurch gekennzeichnet dass** es zwischen 0,05 % und 100 % im Gewicht, vorzugsweise zwischen 1 % und 10 % im Gewicht wenigstens einen, aus der Mandel des Kerns der Frucht des Mirabellenbaums gewonnenen Lipidextrakt enthält, als ein aktives Mittel zum Weichmachen, Feuchtigkeitsspenden und um die Frische des Teints und dessen strahlenden Glanz zu verbesseren.

7. Kosmetisches Erzeugnis für den örtlich wirkenden (topischen) Gebrauch für Phanere, besonders für das Haar, **dadurch gekennzeichnet dass** es zwischen 0,05 % und 100 % im Gewicht und vorzugsweise zwischen 1 % und 10 % im Gewicht mindestens einen, aus der Mandel des Kerns der Frucht des Mirabellenbaums gewonnenen Lipidextrakt enthält, als aktives Mittel zum Weichmachen, Feuchtigkeitsspenden und um den Glanz des Haars zu verbessern und ein leichteres Entwirren und Frisieren zu ermöglichen.

## Claims

1. Use of at least one lipid extract which is obtained from the stones of the fruits of the mirabelle tree as an agent for preparation of a cosmetic product for locally acting (topical) use in the area of the care of the skin, lips, mucous membranes and/or appendages of the skin.

2. Use as claimed in claim 1, wherein the lipid extract consists of oil or of one lipid fraction or lipid fractions which were obtained from the seeds (almonds) of the stones of the fruits of the mirabelle tree by a process which was chosen in the group formed by extraction by hot or cold pressure, extraction by solvents and extraction by supercritical CO₂.

3. Use as claimed in claim 1 or 2, wherein at least one of the indicated lipid extracts is added to the cosmetic product in raw, refined, deodorized or refined and deodorized form.

4. Use as claimed in one of claims 1 to 3, wherein at least one indicated lipid extract is added to a cosmetic product for the skin, lips and/or mucous membranes as agents for softening and moisturizing and for improving the freshness and radiant glow of the complexion.

5. Use as claimed in one of claims 1 to 3, wherein at least the indicated lipid extract is added to a cosmetic product for the extremities or appendages of the skin and especially for the hair as an agent for softening, moisturizing and improving the sheen anf for easier detangling and styling of the hair.

6. Cosmetic product for topical use for locally acting (topical) use for the skin, the mucous membranes and/or the lips, wherein it contains between 0,05 % and 100 % by weight, preferably between 1 % and 10 % by weight of at least one lipid extract obtained from the seed (almond) of the stone of the fruit of the mirabelle tree as an active agent for softening, moisturizing and improving the freshness of the complexion and its radiant glow.

7. Cosmetic product for topical use for locally acting (topical) use for the extremities or appendages of the skin, especially the hair, wherein it contains between 0,05 % and 100 % by weight, preferably between 1 % and 10 % by weight of at least one lipid extract obtained from the seed (almond) of the stone of the fruit of the mirabelle tree as an active agent for softening, moisturizing and improving the sheen of the hair and enabling easier detangling and styling.

## Revendications

1. Utilisation d'au moins un extrait lipidique de noyaux de fruits du mirabellier, en tant qu'agent pour la préparation d'un produit cosmétique à usage local (topique) pour les soins de la peau, des lèvres, des muqueuses et/ou des phanères.

2. Utilisation selon la revendication 1, **caractérisée en ce que** l'extrait lipidique consiste en de l'huile ou une ou des fractions lipidiques extraite(s) des amandes des noyaux des fruits du mirabellier par un procédé choisi dans le groupe formé par l'extraction par pression à chaud ou à froid, l'extraction par solvant et l'extraction par CO₂ supercritique.

3. Utilisation selon la revendication 1 ou 2, **caractérisée en ce que** ledit au moins un extrait lipidique est intégré dans le produit cosmétique sous forme brute, raffinée, désodorisée ou raffinée et désodorisée.

4. Utilisation selon l'une quelconque des revendications 1 à 3, **caractérisée en ce que** ledit au moins un extrait lipidique est intégré dans un produit cosmétique pour la peau, les lèvres et/ou les muqueuses à titre d'agent émollient, hydratant et améliorant la fraîcheur et l'éclat du teint.

5. Utilisation selon l'une quelconque des revendications 1 à 3, **caractérisée en ce que** ledit au moins un extrait lipidique est intégré dans un produit cosmétique pour les phanères, plus particulièrement les cheveux, à titre d'agent émollient, hydratant, améliorant la brillance et facilitant le démêlage et le coiffage.

6. Produit cosmétique à usage local (topique) pour la peau, les muqueuses et/ou les lèvres, **caractérisé en ce qu'**il comporte entre 0,05 % et 100 % en poids, préférentiellement entre 1 % et 10 % en poids, d'au moins un extrait lipidique de l'amande du noyau du fruit du mirabellier, en tant qu'agent actif émollient, hydratant et améliorant la fraîcheur et l'éclat du teint.

7. Produit cosmétique à usage local (topique) pour les phanères, plus particulièrement les cheveux, **caractérisé en ce qu'**il comporte entre 0,05 % et 100 % en poids, préférentiellement entre 1 % et 10 % en poids, d'au moins un extrait lipidique de l'amande du noyau du fruit du mirabellier, en tant qu'agent actif émollient, hydratant, améliorant la brillance et facilitant le démêlage et le coiffage.
